# EUROPÄISCHE PATENTSCHRIFT

(11) **EP 3 586 756 B1**
(45) Veröffentlichungstag und Bekanntmachung des Hinweises auf die Patenterteilung: **18.05.2022**
(21) Anmeldenummer: 19179697.8
(22) Anmeldetag: 12.06.2019
(51) Int. Cl.: A61B 6/12, G03B 42/02, G03B 42/08, A61B 6/00, A61B 6/14

(54) **RÖNTGENSPEICHERFOLIENSYSTEM**
X-RAY IMAGING PLATE SYSTEM
SYSTÈME D'IMAGERIE À PLAQUES À RAYONS X

(30) Priorität: 15.06.2018 DE 102018114460
(43) Veröffentlichungstag der Anmeldung: 01.01.2020
(73) Patentinhaber: Dürr Dental SE, 74321 Bietigheim-Bissingen (DE)
(72) Erfinder: Weber, Michael, 71576 Burgstetten (DE); Philipps, Bernd, 74199 Untergruppenbach (DE)
(74) Vertreter: Ostertag & Partner Patentanwälte mbB

(56) Entgegenhaltungen:
- DE-A1-102014 014 056
- US-A- 5 202 911
- US-A1- 2017 296 131

## Beschreibung

### 1. Gebiet der Erfindung

Die Erfindung betrifft ein Röntgenspeicherfoliensystem nach Anspruch 1.

### 2. Stand der Technik

Röntgenspeicherfoliensysteme werden in der Industrie, der Medizin und/oder in der Dentalmedizin dazu eingesetzt, um zeitnah Röntgenaufnahmen von einem bestimmten Untersuchungsbereich, wie beispielsweise den Zähnen in einem Kiefer, für den Benutzer zu erzeugen. Dazu werden Röntgenspeicherfolien bezüglich einer Röntgenquelle hinter dem zu röntgenden Untersuchungsbereich, beispielsweise dem Kiefer, angeordnet, und mit Röntgenlicht aus der Röntgenquelle belichtet. Die Speicherfolie trägt dann ein latentes Bild, das mit dem Speicherfolienauslesegerät ausgelesen und als Röntgenaufnahme vergleichbar mit herkömmlichen Röntgenfilmaufnahmen auf einem Anzeigegerät dargestellt wird.

Die derzeit üblichen Röntgenspeicherfoliensysteme verwenden dazu als Speicherfolien sogenannte Photolumineszenz-Speicherfolien, die im Speicherfolienauslesegerät durch Aktivierungslicht punkt- oder linienweise abgescannen und ausgelesen werden. Daher werden derartige Speicherfolienauslesegeräte auch kurz als Scanner bezeichnet. Besonders in der Dentalmedizin werden dabei Durchlaufscanner verwendet, bei welchen Röntgenspeicherfolien ohne Kassettenhülle in einer Richtung durch das Gerät transportiert, dabei gescannt und anschließend gelöscht werden.

Grundsätzlich können derartige Speicherfolien vom Röntgenlicht von der einen oder der anderen Seite der Speicherfolie belichtet werden, da das Röntgenlicht die Speicherfolie vollständig durchdringt. Dennoch haben viele Speicherfolien unterscheidbare Vorder- und Rückseiten, da häufig ein einseitiges Auslesen der Speicherfolien präferiert wird. Insbesondere weisen die Speicherfolien häufig rückseitig eine Schutzbeschichtung auf, welche beispielsweise aus Transportgründen im Speicherfolienauslesegerät an bestimmte Reibräder oder Bandantriebe angepasst ist. Diese Beschichtungen weisen jedoch im Hinblick auf das Röntgenlicht nur minimalste Abschwächungswirkung auf.

Die Speicherfolien besitzen somit eine bevorzugte Vorderseite und eine Rückseite.

Wie in der US 7,140,769 B2 beschrieben, kann es im Klinikalltag vorkommen, dass insbesondere dentale Speicherfolien, welche nicht in einer Kassette transportiert werden, bei der Belichtung versehentlich mit ihrer Rückseite anstatt mit ihrer Vorderseite zur Röntgenquelle angeordnet werden. Dies führt zu Spiegelungen der Bildinformationen in der Röntgenaufnahme.

Es ist daher wünschenswert, dass man bei einer späteren Auswertung der Röntgenaufnahme erkennen kann, von welcher Seite die Speicherfolie belichtet wurde.

Hierfür ist es wie ebenfalls in der US 7,140,769 B2 beschrieben bekannt Abschattungsmarker zu verwenden, welche in einem Aufnahmebereich der Speicherfolie einseitig auf der Speicherfolie aufgebracht sind. Diese Abschattungsmarker bestehen aus einem Material mit einem hohen Wirkungsquerschnitt für Röntgenlicht und schatten das Röntgenlicht, wenn die Belichtung von der Seite des Abschattungsmarkers erfolgt, stark ab. Dadurch ist der Abschattungsmarker bei der Betrachtung der Röntgenaufnahme im einen Fall sichtbar und im anderen Fall nicht sichtbar.

Darüber hinaus ist auch bekannt, die Abschattungsmarker asymmetrisch auszugestalten, um weiter Rückschlüsse auf die Orientierung der Speicherfolie zur Röntgenquelle bzw. möglicherweise stattgefundene Bildtransformationen innerhalb des Auslesegeräts zu erkennen.

Ein ähnlicher Abschattungsmarker ist aus der US 5,202,911 A bekannt.

Nachteilig an diesen bisherigen Abschattungsmarkern ist jedoch, dass bei einer Belichtung der Speicherfolie von der Seite des Abschattungsmarkers in der Röntgenaufnahme ein Teilbereich des Untersuchungsbereichs verdeckt wird und damit einer Diagnose nicht zugänglich ist.

Daher ist aus der DE 10 2014 014 056 A1 ein Abschattungsmarker bekannt, dessen Röntgenabsorption so gering ist, dass das Röntgenbild an der Position des Markers noch ausreichend Informationen über das zu diagnostizierende Objekt enthält.

Ferner ist aus US 2017/0296131 A1 ein Verfahren zur Korrektur von Röntgenbildern durch Transformation in den Ortsfrequenzraum bekannt.

### ZUSAMMENFASSUNG DER ERFINDUNG

Aufgabe der Erfindung ist es daher, ein Röntgenspeicherfoliensystem eingangs genannter Art anzugeben, welches hinsichtlich des Abschattungsmarkers verbessert ist, insbesondere es erlaubt, die Belichtungsrichtung zu erkennen, ohne dass Bildinformationen verloren gehen, und welches es erlaubt, die Röntgenspeicherfolien zu individualisieren.

Erfindungsgemäß wird dies durch ein Röntgenspeicherfoliensystem eingangs genannter Art gelöst, bei welchem
c) der Abschattungsmarker zumindest ein Teil eines an der Speicherfolie angebrachten Funkmarkers ist.

Die Erfinder haben erkannt, dass es im Gegensatz zu den bekannten Systemen nicht notwendig ist, einen Abschattungsmarker mit einer hohen Abschattungswirkung zu versehen, welche bewirkt, dass der Abschattungsmarker in der letztlich dargestellten Röntgenaufnahme für den Benutzer bei normaler Betrachtung direkt sichtbar ist. Stattdessen schlagen die Erfinder vor, einen Abschattungsmarker mit einer derart geringen Abschattungswirkung vorzusehen, dass der Abschattungsmarker in der eigentlichen Röntgenaufnahme, die dem Benutzer üblicherweise dargestellt wird, nicht oder allenfalls nur schwach sichtbar ist.

Insbesondere soll die Röntgenaufnahme durch die geringe Abschattungswirkung diagnostizierbar bleiben. D.h. die für die jeweilige Diagnose interessierenden Strukturen sollen erkennbar bleiben. Dies ist vor allem vorteilhaft, wenn zu untersuchende Strukturen sich in dem Teil des Aufnahmebereichs befinden, in welchem auch der Abschattungsmarker angeordnet ist.

Unter Bildartefakten wird dabei auch verstanden, dass von dem Abschattungsmarker nur ein Teil sichtbar ist, da andere Teile von zu untersuchenden Strukturen überlagert werden.

Durch einen solchen Abschattungsmarker wird somit die Röntgenaufnahme an den abgeschatteten Stellen nur geringfügig in ihrem Dynamikbereich eingeschränkt.

Um dennoch festzustellen, von welcher Seite die Speicherfolie belichtet wurde ist im Auslesegerät ein Erkennungsalgorithmus vorgesehen, welcher speziell dazu eingerichtet ist, die durch den Abschattungsmarker verursachte nur geringe Abschattungen zu erkennen und die Belichtungsseite entsprechend zu bestimmen.

Der Erkennungsalgorithmus kann dazu beispielsweise nach einem gegebenenfalls vorhandenen Bildartefakt wie beispielsweise einem schwachen Schatten suchen.

Derartige Bildartefakte verhindern jedoch nicht die Erkennbarkeit der eigentlichen Strukturen des Untersuchungsbereiches, da die Abschattungswirkung so gering ist.

Da die Abschattungswirkung generell vom verwendeten Markermaterial und Belichtungsparametern wie beispielsweise Röntgenspannung, Röntgenintensität, Signal-Rausch-Verhältnis der Röntgenaufnahme usw. abhängt, sind verschiedenste genaue Ausgestaltungen des Abschattungsmarkers möglich, die jeweils von den zu erwartenden Belichtungsparametern und den zu erwartenden Untersuchungsbereichen abhängen. Für jeden Anwendungsfall kann jedoch mit den erfindungsgemäßen Überlegungen eine geeignete Auswahl getroffen werden, um einerseits eine nur schwache Sichtbarkeit bei normaler Betrachtung und andererseits eine ausreichende Robustheit des Erkennungsalgorithmus zu erreichen.

Unter dem Speicherfolienauslesegerät wird im Rahmen der Erfindung im Zusammenhang mit dem Erkennungsalgorithmus selbstverständlich sowohl ein Speicherfolienauslesegerät verstanden, bei welchem der Erkennungsalgorithmus direkt in einer für das Auslesen separaten Scanner-Einheit integriert ist, als auch ein Speicherfolienauslesegerät, welches eine entsprechende Auswertesoftware mit dem Erkennungsalgorithmus in einem separaten Datenverarbeitungsgerät, wie einem handelsüblichen PC, umfasst. Auch kommt es dabei nicht darauf an, ob der Erkennungsalgorithmus in Hardware oder in Software oder gar als ausgelagerter Dienst auf einem Server implementiert ist.

Unter Röntgenaufnahme wird hier jegliche Repräsentation der Röntgenintensitätsverteilung verstanden. Meist sind die gemessenen Intensitätswerte in einem zweidimensionalen Array abgespeichert oder bereits als Grauwerte hinterlegt.

Erfindungsgemäß ist vorgesehen, dass der Abschattungsmarker zumindest ein Teil eines an der Speicherfolie angebrachten Funkmarkers ist.

Funkmarker, insbesondere RFID-Marker, werden in der Technik häufig eingesetzt, um eine Vielzahl von Gegenständen zu individualisieren, indem den Gegenständen eine eindeutige ID zugeordnet wird, die sich über Funk auslesen lässt. Dies kann auch bei Speicherfolien vorteilhaft sein, da die jeweilige Speicherfolie dann direkt oder indirekt mithilfe einer entsprechenden Datenbank bestimmten Belichtungsvorgängen und/oder Patienten zuordenbar ist. Auch können die Funkmarker Ausleseparameter bestimmen, die zu einer optimalen Auslesung im Speicherfolienauslesegerät führen. Dazu können an der Röntgenbelichtungseinrichtung sowie an dem Speicherfolienauslesegerät entsprechende Funk-Lese- und/oder Schreibgeräte vorgesehen sein.

Aus Produktionsgründen ist es daher vorteilhaft, einen ohnehin auf einer Seite der Röntgenspeicherfolie vorgesehenen Funkmarker zugleich als Abschattungsmarker zu verwenden.

Vorzugsweise ist vorgesehen, dass der Abschattungsmarker eine geometrische Form mit einem charakteristischen Merkmal hat und dass der Erkennungsalgorithmus das charakteristische Merkmal verwendet, um eine mögliche Abschattungswirkung zu erkennen.

Dadurch kann der Erkennungsalgorithmus zielgerichtet nach der Abschattungswirkung in dem ausgewerteten Bild suchen, und ermitteln, ob eine Abschattungswirkung, d. h. eine entsprechende Belichtung seitens des Abschattungsmarkers, erfolgt ist oder nicht. Sofern das charakteristische Merkmal nicht auch in dem zu röntgenden Untersuchungsbereich vorhanden ist, kann somit eine zuverlässige Erkennung stattfinden. Ein charakteristisches Merkmal kann beispielsweise eine Abmessung des Abschattungsmarkers sein.

Vorzugsweise ist vorgesehen, dass der Abschattungsmarker eine definierte Periodizität aufweist und der Erkennungsalgorithmus die Röntgenaufnahme im Hinblick auf die Periodizität auswertet.

Wird das Röntgenspeicherfoliensystem in der Medizin oder der Dentalmedizin eingesetzt, treten im Untersuchungsbereich typischerweise keine periodische Struktur auf. Daher kann ein Abschattungsmarker mit einer vorbekannten Periodizität beispielsweise in seiner geometrischen Form und/oder seiner Abschattungswirkung vom Erkennungsalgorithmus zuverlässig erkannt werden.

Vorzugsweise ist vorgesehen, dass der Erkennungsalgorithmus die Röntgenaufnahme von einem Ortsraum in einen Frequenzraum überführt und im Frequenzraum nach einer zu der Periodizität des Abschattungsmarkers gehörigen Frequenz sucht.

Eine Periodizität kann im Frequenzraum besonders gut erkannt werden. Eine Möglichkeit stellt hierbei die Überführung in den Fourier-Raum dar, beispielsweise über die Diskrete Fourier-Transformation (DFT).

Aufgrund des Zusammenhangs zwischen Orts- und Frequenzraums und entsprechenden Bildauswertungsfiltern kann eine derartige Periodizität des Abschattungsmarkers aber auch mit anderweitigen Bildfiltern, die gegebenenfalls direkt im Ortsraum arbeiten, stattfinden.

Vorzugsweise ist vorgesehen, dass der Erkennungsalgorithmus bei der Erkennung der Abschattung auf Informationen zugreift, die jeweils dem individuellen Funkmarker verknüpft sind.

Beispielsweise kann eine Datenbank ein charakteristisches Merkmal des jeweiligen Funkmarkers beinhalten, sodass nach Auslesen der Funkmarker-Informationen der Erkennungsalgorithmus gezielt nach den relevanten Abschattungsmerkmalen suchen kann.

So wird beispielsweise die Abschattungserkennung an unterschiedlichen Speicherfoliengrößen verbessert oder ein Wechsel der verwendeten Funkmarker auf anders gestaltete Funkmarker im Rahmen einer Produktpflege ermöglicht, indem der Erkennungsalgorithmus die entsprechenden Informationen erhält.

Vorzugsweise ist vorgesehen, dass der Abschattungsmarker eine Antennenstruktur des Funkmarkers ist.

Eine solche Antennenstruktur kann beispielsweise die Antenne eines RFID-Markers sein.

Vorzugsweise ist vorgesehen, dass die Antennenstruktur des Funkmarkers eine Spiralform aufweist und der Erkennungsalgorithmus ein charakteristische Merkmal der Spiralform in der Röntgenaufnahme sucht und daraus bestimmt, ob eine Abschattungswirkung vorliegt oder nicht.

Eine spiralförmige Antennenstruktur weist zwischen den Spiralarmen in etwa gleiche Abstände auf, die sich mehrfach wiederholen, sodass eine Periodizität vorhanden ist. Diese Periodizität kann im Frequenzraum wiederum sehr gut erkannt werden.

Vorzugsweise ist vorgesehen, dass der Abschattungsmarker aus einem elektrisch leitfähigen Material ausgebildet ist.

Dies ermöglicht es, den Abschattungsmarker zugleich für andere Zwecke wie beispielsweise eine Antennenstruktur zu verwenden.

Dazu kann der Abschattungsmarker beispielsweise aus Kupfer ausgebildet sein. Er kann aber auch aus einem Leichtmetall, insbesondere Aluminium, ausgebildet sein. Ein Abschattungsmarker aus Leichtmetall bewirkt nur eine geringe Abschattung des Röntgenlichts, da dessen Röntgenwechselwirkungsquerschnitt klein ist.

Vorzugsweise ist vorgesehen, dass der Abschattungsmarker mit Hilfe eines Druckverfahrens als Druckfarbe auf die Röntgenspeicherfolie aufgedruckt ist.

Ein Druckverfahren, wie beispielsweise Siebdruck, stellt einen einfachen Fertigungsprozess für den Abschattungsmarker auf einer Röntgenspeicherfolie dar. Für die Abschattungswirkung kann die Druckfarbe beispielsweise Bariumsulfat aufweisen.

Vorzugsweise ist vorgesehen, dass der Abschattungsmarker eine Abschattungswirkung von weniger als etwa 20%, vorzugsweise weniger als etwa 10%, insbesondere weniger als etwa 5%, der auftreffenden Röntgenintensität bewirkt.

Eine derart geringe Abschattungswirkung stellt sicher, dass der Abschattungsmarker bei einer Betrachtung, bei der die interessierenden Strukturen des zu röntgenden Untersuchungsbereichs für den Benutzer noch erkennbar sind, gerade nicht sichtbar ist.

Vorzugsweise ist vorgesehen, dass der Abschattungsmarker eine Abschattungswirkung von mehr als etwa 1%, vorzugsweise mehr als etwa 2%, insbesondere mehr als etwa 4%, der auftreffenden Röntgenintensität bewirkt.

Eine Untergrenze für die Abschattungswirkung garantiert eine ausreichende Robustheit des Erkennungsalgorithmus, um zweifelsfrei festzustellen, ob eine Fehlbelichtung vorliegt oder nicht.

### KURZE BESCHREIBUNG DER ZEICHNUNGEN

Nachfolgend werden Ausführungsbeispiele der Erfindung anhand der Zeichnungen näher erläutert. In diesen zeigen:
- Figur 1: eine schematische Darstellung eines Röntgenspeicherfoliensystems mit einem Abschattungsmarker;
- Figur 2: eine Darstellung einer Röntgenaufnahme die dem Benutzer bei normaler Betrachtung gezeigt wird, wobei die Belichtung seitens des Abschattungsmarkers erfolgte;
- Figur 3: ein Frequenzraumbild dieser Röntgenaufnahme nach einer Transformation der Röntgenaufnahme in den Frequenzraum;
- Figur 4: eine Darstellung der Röntgenaufnahme aus Figur 2, die aufgrund der Belichtung seitens des Abschattungsmarkers gespiegelt wurde und einen informativen Hinweis zur Belichtungsseite zeigt.

### BESCHREIBUNG BEVORZUGTER AUSFÜHRUNGSBEISPIELE

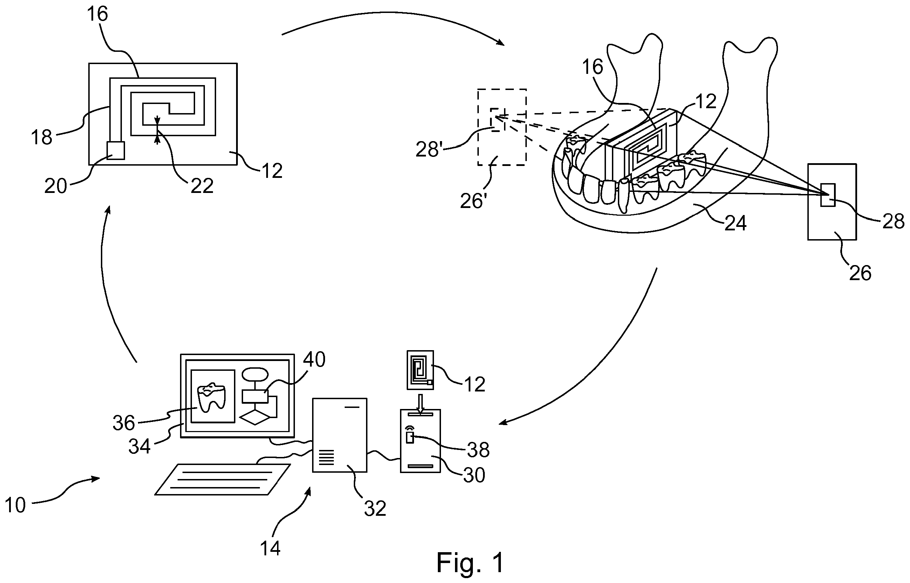

Figur 1 zeigt ein insgesamt den typischen Zyklus einer Röntgenaufnahme im Dentalmedizinbereich mit einem im unteren Teil der Figur 1 dargestellten Röntgenspeicherfolienauslesesystem 10.

Das Röntgenspeicherfolienauslesesystem 10 umfasst eine Röntgenspeicherfolie 12 sowie ein Speicherfolienauslesegerät 14.

Wie links oben aus Figur 1 ersichtlich, weist die Röntgenspeicherfolie 12 einen Abschattungsmarker 16 auf, der auf einer Seite der Röntgenspeicherfolie 12, typischerweise deren Rückseite, angeordnet ist. Der Abschattungsmarker 16 ist zumindest auch in einem Aufnahmebereich der Röntgenspeicherfolie 12 angeordnet, welcher für die eigentliche Belichtung vorgesehen ist. Der Abschattungsmarker 16 weist gegenüber vorbekannten Abschattungsmarkern 16 beispielsweise aus Kupfer jedoch nur eine geringe Abschattungswirkung auf.

Der Abschattungsmarker 16 ist im hier gezeigten Ausführungsbeispiel als spiralförmige Antennenstruktur 18 eines RFID-Markers 20 ausgebildet, welcher die Röntgenspeicherfolie 12 zur leichteren Zuordnung gegenüber anderer Röntgenspeicherfolien 12 des Röntgenspeicherfolienauslesesystems 10 individualisiert.

Die Spiralform der Antennenstruktur 18 hat einen durch die elektromagnetischen Eigenschaften vorgegebenen im Wesentlichen entlang der Spiralform gleichbleibenden Abstand 22 zwischen den einzelnen Aluminium-Leiterbahnen.

Mit dem gleichbleibenden Abstand 22 hat der Abschattungsmarker 16 im hier gezeigten Ausführungsbeispiel als charakteristisches Merkmal sich periodisch abwechselnde Bereiche von Aluminium-Leiterbahnen und Zwischenräumen zwischen den Leiterbahnen.

Wie im rechten oberen Teil der Figur 1 gezeigt, wird die Röntgenspeicherfolie 12 zur Belichtung in den Innenraum eines Kiefers 24 eingebracht und mit einer nicht zwingend zum Röntgenspeicherfoliensystem 10 gehörigen Röntgenbelichtungseinrichtung 26 und einer darin angeordneten Röntgenquelle 28 belichtet.

Dabei zeigt die den Abschattungsmarker 16 tragende Rückseite der Röntgenspeicherfolie 12 zur Röntgenquelle 28, sodass die Belichtung seitens des Abschattungsmarkers 16 erfolgt. Dies entspricht einer versehentlich nicht richtig eingesetzten Röntgenspeicherfolie 12.

Zum besseren Verständnis der unterschiedlichen Belichtungsrichtungen ist ferner gestrichelt eine komplementäre Position der Röntgenbelichtungseinrichtung 26' und der Röntgenquelle 28' gezeigt.

Nach der Belichtung der Röntgenspeicherfolie 12 wird diese in das Speicherfolienauslesegerät 14 eingebracht. Das Speicherfolienauslesegerät weist zum Auslesen der Speicherfolie 12 die von derartigen Speicherfolienauslesegeräten 14 bekannten Komponenten in einer Scanner-Einheit 30 auf.

Die Scanner-Einheit 30 ist hier als Durchlaufscanner ausgebildet, kann jedoch ein beliebiges Scanverfahren verwenden, das zur Photolumineszenz-Erfassung von Röntgenspeicherfolien 12 geeignet ist.

Ferner weist die Scanner-Einheit 30 ein RFID-Lesegerät 38 auf, mit welchem die Informationen des RFID-Markers 20 per Funk ausgelesen werden.

Die ausgelesenen Daten der Röntgenaufnahme sowie gegebenenfalls die Informationen des RFID-Markers 20 werden daraufhin an eine Auswerteeinheit 32 übergeben, die hier als handelsüblicher PC dargestellt ist.

Auf einem Anzeigegerät 34 der Auswerteeinheit 32 kann ein übliches Grauwertbild 36 der Röntgenaufnahme dargestellt werden. In diesem Grauwertbild 36 ist für den Benutzer bei normaler Betrachtung jedoch der Abschattungsmarker 16 aufgrund seiner geringen Abschattungswirkung nicht erkennbar.

Die Auswerteeinheit 32 weist daher einen Erkennungsalgorithmus 40 auf, der dazu eingerichtet ist, zu erkennen, von welcher Seite die Röntgenspeicherfolie 12 belichtet wurde, indem nach einer Abschattungswirkung des Abschattungsmarker 16 in der Röntgenaufnahme gesucht wird.

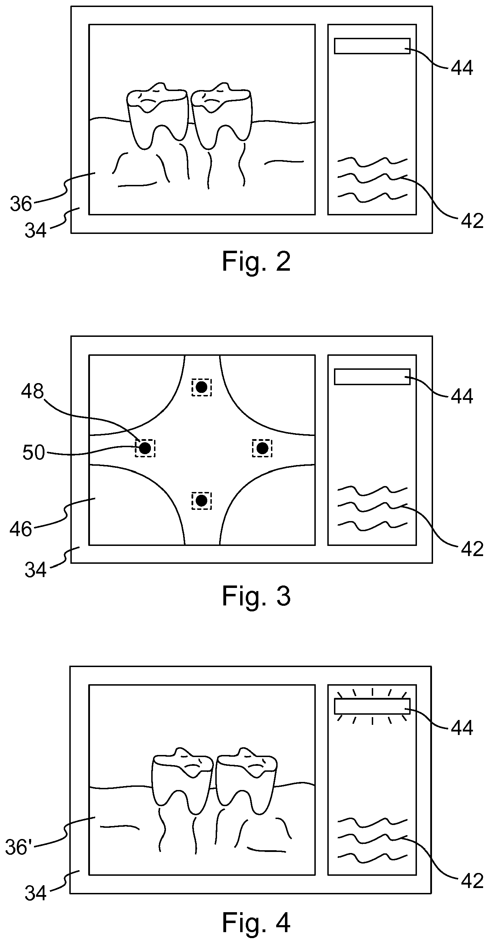

Der Erkennungsalgorithmus 40 arbeitet wie folgt:
Als Eingangsinformation erhält der Erkennungsalgorithmus 40 die Röntgenaufnahme, die als Grauwertbild 36, welches in Figur 2 nochmals gezeigt ist, dem Benutzer dargestellt würde. Wie aus Figur 2 ersichtlich, werden neben und/oder über dem Grauwertbild 36 Patienteninformationen 42 dargestellt. Ferner ist ein Fehlbelichtungshinweis 44 vorgesehen, der anzeigt, von welcher Seite die Röntgenspeicherfolie 12 belichtet wurde.

Wie auch aus Figur 2 nochmals ersichtlich ist, lassen sich aus diesem Grauwertbild 36, das für die normale Betrachtung durch einen Bediener vorgesehen ist, durch den Benutzer optisch keine Rückschlüsse auf das Vorhandensein der Abschattungswirkung des Abschattungsmarker 16 ziehen.

Der Erkennungsalgorithmus 40 überführt nun die Röntgenaufnahme von einem Ortsraum in den Frequenzraum, indem die Bilddaten einer Fourier-Transformation unterzogen werden. Eine Darstellung des entsprechenden Frequenzraumbildes 46 könnte in etwa wie in Figur 3 gezeigt aussehen.

Aufgrund der Periodizität der Antennenstruktur 18 mit dem Abstand 22 bilden sich wie in Figur 3 ersichtlich an durch die Periodizität definierten Positionen 48 des Frequenzraumbildes 46 Intensitätspeaks 50 heraus.

Der Erkennungsalgorithmus 40 sucht daher an den vorbekannten Positionen 48 nach dem Vorhandensein derartiger Intensitätspeaks 50.

Die vorbekannten Positionen 48 können dabei in Abhängigkeit der vom RFID-Marker 20 erhaltenen Informationen zur Röntgenspeicherfolie 12 und damit auch zu dem darauf angebrachten Abschattungsmarker 16 bestimmt werden.

Durch die Anwendung von Entscheidungskriterien bestimmt der Erkennungsalgorithmus 40 somit aufgrund dessen, dass im vorliegenden Beispiel die Intensitätspeaks 50 vorhanden sind, dass eine Belichtung der Röntgenspeicherfolie seitens des Abschattungsmarkers 16 vorliegt.

Der Erkennungsalgorithmus 40 kann daher wie in Figur 4 gezeigt, den Fehlbelichtungshinweis 44 aktivieren, aus welchem deutlich wird, dass eine Belichtung seitens des Abschattungsmarkers 16 erfolgte.

Ferner kann nun aufgrund der Erkennung der Abschattungswirkung die Röntgenaufnahme gespiegelt werden, sodass das in Figur 4 gezeigte Grauwertbild 36' die richtige Orientierung des Kiefers 24 zeigt.

Zusätzlich kann der Erkennungsalgorithmus 40 auch einen Abschattungsmarker-Unterdrückungsalgorithmus aufweisen, welcher die durch die Abschattungswirkung hervorgerufenen Effekte wie beispielweise einen leichten Hintergrundschatten negiert.

Selbstverständlich kann der Erkennungsalgorithmus 40 auf die Darstellung der Zwischenschritte verzichten, sodass dem Benutzer letztlich nur das endgültige Grauwertbild 36' angezeigt wird.

## Patentansprüche

1. Röntgenspeicherfoliensystem (10) mit
a) einer Röntgenspeicherfolie (12), die dazu eingerichtet ist, um in einem Aufnahmebereich mit Röntgenlicht belichtet zu werden, und die zumindest auf einer Seite einen Abschattungsmarker (16) trägt, der im Aufnahmebereich angeordnet ist,
b) einem Speicherfolienauslesegerät (14), das dazu eingerichtet ist, die belichtete Röntgenspeicherfolie (12) auszulesen, um eine Röntgenaufnahme (36, 36') zu erzeugen,
wobei
c) der Abschattungsmarker (16) eine Abschattungswirkung gegen Röntgenlicht aufweist, die derart gering ist, insbesondere bei maximal etwa 30% der auftreffenden Röntgenintensität liegt, dass der Abschattungsmarker (16) bei Betrachtung der Röntgenaufnahme (36, 36') durch den Benutzer nur schwach, nur durch Bildartefakte und/oder nicht erkennbar ist, und wobei
d) das Speicherfolienauslesegerät (14) einen Erkennungsalgorithmus (40) aufweist, der dazu eingerichtet ist, zu erkennen, ob das Röntgenlicht bei der Belichtung vom Abschattungsmarker (16) abgeschattet wurde oder nicht,
**dadurch gekennzeichnet, dass**
e) der Abschattungsmarker (16) zumindest ein Teil eines an der Röntgenspeicherfolie (12) angebrachten Funkmarkers (20) ist.

2. Röntgenspeicherfoliensystem nach Anspruch 1, **dadurch gekennzeichnet, dass** der Abschattungsmarker (16) eine geometrische Form mit einem charakteristischen Merkmal (22) hat und dass der Erkennungsalgorithmus (40) das charakteristische Merkmal (22) verwendet, um eine mögliche Abschattungswirkung zu erkennen.

3. Röntgenspeicherfoliensystem nach einem der vorhergehenden Ansprüche, **dadurch gekennzeichnet, dass** der Abschattungsmarker (16) eine definierte Periodizität (22) aufweist und der Erkennungsalgorithmus (40) die Röntgenaufnahme (36, 36') im Hinblick auf die Periodizität (22) auswertet.

4. Röntgenspeicherfoliensystem nach Anspruch 3, **dadurch gekennzeichnet, dass** der Erkennungsalgorithmus (40) die Röntgenaufnahme (36, 36') von einem Ortsraum in einen Frequenzraum (46) überführt und im Frequenzraum (46) nach einer zu der Periodizität (22) des Abschattungsmarkers (16) gehörigen Frequenz (50) sucht.

5. Röntgenspeicherfoliensystem nach einem der vorhergehenden Ansprüche, **dadurch gekennzeichnet, dass** der Abschattungsmarker (16) eine Antennenstruktur (18) des Funkmarkers (20) ist.

6. Röntgenspeicherfoliensystem nach Anspruch 5, **dadurch gekennzeichnet, dass** die Antennenstruktur (18) des Funkmarkers (20) eine Spiralform aufweist und der Erkennungsalgorithmus (40) ein charakteristische Merkmal (22) der Spiralform in der Röntgenaufnahme (36, 36', 46) sucht und daraus bestimmt, ob eine Abschattungswirkung vorliegt oder nicht.

7. Röntgenspeicherfoliensystem nach einem der vorhergehenden Ansprüche, **dadurch gekennzeichnet, dass** der Abschattungsmarker (16) aus einem elektrisch leitfähigen Material ausgebildet ist.

8. Röntgenspeicherfoliensystem nach einem der vorhergehenden Ansprüche, **dadurch gekennzeichnet, dass** der Abschattungsmarker (16) mit Hilfe eines Druckverfahrens als Druckfarbe auf die Röntgenspeicherfolie (12) aufgedruckt ist.

9. Röntgenspeicherfoliensystem nach einem der vorhergehenden Ansprüche, **dadurch gekennzeichnet, dass** der Abschattungsmarker (16) eine Abschattungswirkung von weniger als etwa 20%, vorzugsweise weniger als etwa 10%, insbesondere weniger als etwa 5%, der auftreffenden Röntgenintensität bewirkt.

## Claims

1. X-ray imaging plate system (10) comprising
a) an x-ray imaging plate (12) which is configured to be exposed by x-ray light in a recording region and which on at least one side carries a shadowing marker (16), which is arranged in the recording region,
b) an imaging plate reader (14) which is configured to read the exposed x-ray imaging plate (12) in order to produce an x-ray recording (36, 36'),
wherein
c) the shadowing marker (16) has a shadowing effect in respect of x-ray light that is so small, in particular no more than approximately 30% of the incident x-ray intensity, that the shadowing marker (16) is only weakly identifiable, only identifiable by way of image artefacts and/or not identifiable when the x-ray recording (36, 36') is observed by the user, and in that
d) the imaging plate reader (14) has an identification algorithm (40) which is configured to identify whether or not the x-ray light has been shadowed by the shadowing marker (16) during the exposure,
**characterized in that**
e) the shadowing marker (16) is at least a part of a radio marker (20) attached to the x-ray imaging plate (12).

2. X-ray phosphor imaging system according to Claim 1, **characterized in that** the shadowing marker (16) has a geometric form with a characteristic feature (22) and **in that** the identification algorithm (40) uses the characteristic feature (22) to identify a possible shadowing effect.

3. X-ray imaging plate system according to any of the preceding claims, **characterized in that** the shadowing marker (16) has a defined periodicity (22) and the identification algorithm (40) analyses the x-ray recording (36, 36') in view of the periodicity (22).

4. X-ray imaging plate system according to Claim 3, **characterized in that** the identification algorithm (40) converts the x-ray recording (36, 36') from real space to frequency space (46) and seeks for a frequency (50) belonging to the periodicity (22) of the shadowing marker (16) in the frequency space (46).

5. X-ray imaging plate system according to any of the preceding claims, **characterized in that** the shadowing marker (16) is an antenna structure (18) of the radio marker (20).

6. X-ray imaging plate system according to Claim 5, **characterized in that** the antenna structure (18) of the radio marker (20) has a spiral form and the identification algorithm (40) seeks for a characteristic feature (22) of the spiral form in the x-ray recording (36, 36', 46) and determines whether or not a shadowing effect is present therefrom.

7. X-ray imaging plate system according to any one of the preceding claims, **characterized in that** the shadowing marker (16) is made of an electrically conductive material.

8. X-ray imaging plate system according to any one of the preceding claims, **characterized in that** the shadowing marker (16) is printed onto the x-ray phosphor plate (12) as printing ink with the aid of a printing method.

9. X-ray imaging plate system according to any one of the preceding claims, **characterized in that** the shadowing marker (16) causes a shadowing effect of less than approximately 20%, preferably of less than approximately 10%, in particular of less than approximately 5%, of the incident x-ray intensity.

## Revendications

1. Système d'imagerie à plaques à rayons X (10) comportant
a) une plaque à rayons X (12), qui est adaptée pour être exposée à la lumière aux rayons X dans une zone de prise de vue, et qui porte au moins sur un côté un marqueur d'ombre (16), qui est agencé dans la zone de prise de vue,
b) un appareil de lecture de plaque à rayons X (14), qui est adapté pour lire la plaque à rayons X (12) exposée pour créer une radiographie (36, 36'),
dans lequel
c) le marqueur d'ombrage (16) comporte un effet d'ombrage contre la lumière aux rayons, qui est si faible, surtout est de l'ordre de 30% maximum environ de l'intensité incidente des rayons X, que le marqueur d'ombrage (16) n'est que faiblement visible, visible uniquement par des artefacts d'image et / ou n'est pas visible lors de l'observation de la radiographie par l'utilisateur, et dans lequel
d) l'appareil de lecture de plaque à rayons X (14) comporte un algorithme de reconnaissance (40), qui est adapté pour reconnaître, si la lumière aux rayons X a été obscurci ou non par le marqueur d'ombrage (16) lors de l'exposition,
**caractérisé en ce que**
e) le marqueur d'ombrage (16) est au moins une partie d'un marqueur radio (20) attaché à la plaque à rayons X (12).

2. Système d'imagerie à plaques à rayons X selon la revendication 1, **caractérisé en ce que** le marqueur d'ombrage (16) a une forme géométrique présentant une caractéristique particulière (22), et que l'algorithme de reconnaissance (40) utilise la caractéristique particulière (22) pour reconnaître un effet d'ombrage possible.

3. Système d'imagerie à plaques à rayons X selon l'une quelconque des revendications précédentes, **caractérisé en ce que** le marqueur d'ombrage (16) comporte une périodicité définie (22), et que l'algorithme de reconnaissance (40) évalue la radiographie (36, 36') au regard de la périodicité (22).

4. Système d'imagerie à plaques à rayons X selon la revendication 3, **caractérisé en ce que** l'algorithme de reconnaissance (40) transfère la radiographie (36, 36') d'un espace local dans un espace de fréquences (46), et cherche dans l'espace de fréquences (46) une fréquence (50) appartenant à la périodicité (22) du marqueur d'ombrage (16).

5. Système d'imagerie à plaques à rayons X selon l'une quelconque des revendications précédentes, **caractérisé en ce que** le marqueur d'ombrage (16) est une structure d'antenne (18) du marqueur radio (20).

6. Système d'imagerie à plaques à rayons X selon la revendication 5, **caractérisé en ce que** la structure d'antenne (18) du marqueur radio (20) comporte une forme en spirale, et que l'algorithme de reconnaissance (40) cherche une caractéristique particulière (22) de la forme en spirale dans la radiographie (36, 36', 46), et détermine à partir de celle-ci, s'il existe ou s'il n'existe pas un effet d'ombrage.

7. Système d'imagerie à plaques à rayons X selon l'une quelconque des revendications précédentes, **caractérisé en ce que** le marqueur d'ombrage (16) est constitué d'un matériau électriquement conducteur.

8. Système d'imagerie à plaques à rayons X selon l'une quelconque des revendications précédentes, **caractérisé en ce que** le marqueur d'ombrage (16) est imprimé sur la plaque à rayons X (12) à l'aide d'un procédé d'impression en tant qu'encre d'impression.

9. Système d'imagerie à plaques à rayons X selon l'une quelconque des revendications précédentes, **caractérisé en ce que** le marqueur d'ombrage (16) provoque un effet d'ombrage de moins d'environ 20 %, de préférence de moins d'environ 10 %, surtout de moins d'environ 5 % de l'intensité incidente des rayons X.
